# EUROPÄISCHE PATENTSCHRIFT

(11) **EP 1 408 833 B1**
(45) Veröffentlichungstag und Bekanntmachung des Hinweises auf die Patenterteilung: **20.12.2006**
(21) Anmeldenummer: 00918655.2
(22) Anmeldetag: 03.05.2000
(51) Int. Cl.: A61B 5/103

(54) **MESSVORRICHTUNG ZUR BESTIMMUNG EINER FEHLBELASTUNG EINES MENSCHLICHEN KÖRPERS IN AUFRECHTER HALTUNG**
MEASURING DEVICE FOR DETERMINING AN UNEVEN LOADING OF THE HUMAN BODY IN THE UPRIGHT POSITION
DISPOSITIF DE MESURE DESTINE A DETERMINER UNE CONTRAINTE IRREGULIERE SUR UN CORPS HUMAIN EN STATION DEBOUT

(30) Priorität: 06.03.2000 CH 431002000
(43) Veröffentlichungstag der Anmeldung: 21.04.2004
(73) Patentinhaber: Morger, Otto, 8640 Rapperswil (CH)
(72) Erfinder: Morger, Otto, 8640 Rapperswil (CH)
(74) Vertreter: Schneider Feldmann AG Patent- und Markenanwälte
(86) Internationale Anmeldenummer: PCT/CH2000/000246
(87) Internationale Veröffentlichungsnummer: WO 2001/066009

(56) Entgegenhaltungen:
- WO-A-95/35063
- DE-A- 19 712 229
- US-A- 5 443 079

## Beschreibung

Die vorliegende Erfindung betrifft eine Messvorrichtung zur Bestimmung einer Fehlhaltung des menschlichen Körpers in aufrechter Haltung gemäss Oberbegriff des Patentanspruches 1.

Menschen mit fehlerhafter aufrechter Haltung belasten ihren Bewegungsapparat ungleichmässig und leiden deshalb an den verschiedensten Körperstellen an Schmerzen. Zur Bestimmung der Fehlbelastung sind gemäss des bekannten Standes der Technik verschiedene Vorrichtungen bekannt.
Sogenannte Hüft-Wasserwaagen bestehen aus einem Grundkörper oder Führungsglied mit zwei daran Schwenkbeweglich angeordneten Schenkeln oder Schnäbeln, in denen jeweils eine Wasserwaage respektive eine Libelle integriert ist. Für die Bestimmung der Körperhaltung muss sich die zu vermessende Person in ihrer natürlichen Haltung aufrecht hinstellen. Die diagnostizierende Person hält ihr dann die Hüft-Wasserwaage von hinten auf der Höhe der Hüfte an den Körper. Die schwenkbaren Schenkel werden dabei beidseitig auf die Hüftknochen aufgelegt. Anhand der Abweichung der Libelle der Wasserwaage von der Horizontalen kann diagnostiziert werden, ob und wie stark der Patient das Becken in einer Schieflage hält. Im allgemeinen wird diese Schieflage als Beinverkürzung bezeichnet, wobei diese tatsächlich in Form eines kürzeren Beines oder im häufigeren Falle nur scheinbar, im allgemeinen verursacht durch eine Ueberdehnung von Muskeln oder Sehnen, vorhanden ist. Ursache einer derartigen scheinbaren Beinverkürzung kann ein Schlag oder eine kurzzeitige Fehlbelastung des Bewegungsapparates sein.
Eine Hüftwasserwaage zur Bestimmung der Beinverkürzung gemäss dem oben beschriebenen Stand der Technik ist aus CH-A-671'330 bekannt.

Die Hüftwasserwaage gemäss dem Stand der Technik birgt mehrere Nachteile. Der Körper gleicht durch Verschiebung des Beckens die schiefe Haltung aus. Die Hüft-Wasserwaage misst deshalb die bereits teilweise kompensierte Schieflage, nicht jedoch die effektive Fehlbelastung des Beckens und der Beine.
Ferner stellt sich in dieser Situation kaum ein Patient in seiner natürlichen Haltung hin, so dass die Messung verfälscht ist. Zudem ist die Lage der Hüftknochen gerade bei korpulenten Patienten nicht einfach feststellbar, so dass die Messung deshalb oft nicht sorgfältig durchgeführt werden kann.

Es sind deshalb mehrere Messvorrichtungen bekannt, welche die Fehlbelastung der Beine direkt bestimmen, indem sich der Patient mit je einem Fuss auf eine Waage stellt.
FR-A-2'491'754 beschreibt eine derartige Vorrichtung zur Bestimmung einer Beinverkürzung. Der Patient stellt sich mit je einem Fuss auf eine vertikal bewegliche Platte, wobei von vorne Anschlagelemente an seine Knie und sein Becken gepresst werden. Die unterschiedliche Gewichtsbelastung der zwei Platten wird registriert und die Platten gegenseitig so angehoben beziehungsweise gesenkt, bis sie dieselbe Gewichtsbelastung registrieren. Der Höhenunterschied der zwei Platten veranschaulicht die Beinverkürzung.

US-A-5'088'504 offenbart eine Messvorrichtung, mit welcher sich eine Beckenverschiebung und die Gewichtsbelastung auf jedes Bein feststellen lässt. Die Messvorrichtung umfasst einen Sockel und eine daran angebrachte Vertikalachse. Im Sockel sind zwei Waagen angeordnet, wobei sie mit einem Anschlag für die Fersen des Patienten versehen sind. An der Vertikalachse ist ein Messelement zur Bestimmung der Beckenverschiebung verschiebbar angeordnet, wobei das Messelement einen Vertikalverschiebestab zur Höheneinstellung und zwei horizontal verschiebbare Anschlaglatten zur Beckenvermessung umfasst. Zur Vermessung stellt sich der Patient freistehend mit dem Rücken zur Vertikalachse auf den Sockel.

In US-A-4'033'329 ist eine ähnliche Messvorrichtung beschrieben, bei welcher jedoch der Positionierungsanschlag für die Fersen fehlt. Eine verlässliche Reproduktion der Messung ist bei fehlendem Referenzpunkt jedoch nicht möglich.

WO-A-95/35063 zeigt eine Messvorrichtung mit zwei Waagen und einer Vertikalachse, an der zwei Mess- und Fixierungselemente, eines für das Becken und eines für die Schultern, angeordnet sind. Für die Vermessung steht der Patient mit dem Gesicht zur Vertikalachse, wobei auf den Waagen Elemente zur Positionierung der Fersen vorhanden sind.

Aus der AT-U-002'239 des Anmelders ist eine Messvorrichtung zur Bestimmung einer Fehlbelastung des menschlichen Körpers bekannt, die ein zwei Waagen aufweisendes Basiselement und eine Justiereinheit zur Positionierung einzelner Körperpunkte oder - bereiche der zu vermessenden Person umfasst. Die Justiereinheit weist eine auf dem Basiselement angeordnete Vertikalachse auf, an der Fixierungselemente vertikal verschiebbar angeordnet sind, wobei ein erstes Fixierungselement zur Fixierung der Hüften und ein zweites Fixierungselement zur Fixierung des Oberkörpers vorhanden ist. Am Basiselement ist ein Anschlag zur Positionierung der Fersen der zu vermessenden Person vorhanden. Die Waagen sind absolut gegenüber einem Eichwert oder relativ zueinander eichbar. Ferner ist ein drittes Fixierungselement vorhanden zur Fixierung der Knie sowie ein seitlicher Anschlag für die Fuss-Seiten. Der Anschlag zur Positionierung der Fersen ist auf der der Vertikalachse zugewandten Seite angeordnet, wobei die mindestens eine Vertikalachse eine Positionierachse zur Definition einer mittleren Körperlinie der zu vermessenden Person bildet.

Alle diese Messvorrichtungen weisen jedoch nach wie vor den Nachteil auf, dass die Messungen nur unzureichende Daten für eine exakte Diagnose liefern, da bei den einfacheren Geräten nicht die Haltung des gesamten Körpers berücksichtigt wird, und bei den weiterentwickelten Geräten die Abweichungen der Körperhaltung auf ein zweidimensionales Muster reduziert werden. Dadurch lassen sich die Fehlhaltungen und Verkürzungen nicht eindeutig bestimmen, da Verdrehungen oder Distorsionen nicht berücksichtigt werden. So führt zum Beispiel eine Distorsion des Beckens zu einer Fehlstellung der Darmbeinschaufel und des Femurs. Da die bisher bekannten Geräte lediglich zweidimensionale Abweichungen innerhalb der Frontalebene erfassen würde eine solche Distorsions-Fehlstellung als Verkürzung respektive Verlängerung des Femurs diagnostiziert, obwohl keinerlei Längenveränderung des Femurs vorliegt. Analoges gilt für Distorsionen des Schultergürtels

Die unzureichende, zweidimensionale Datengrundlage für die Diagnose erlaubt es daher nicht die notwendigen therapeutischen Massnahmen korrekt zu planen und durchzuführen.

Es ist deshalb Aufgabe der Erfindung, eine Messvorrichtung zur Bestimmung einer Fehlbelastung eines menschlichen Körpers in aufrechter Haltung zu schaffen, welche eine einfache und doch eindeutige Bestimmung der Abweichungen in allen Raumrichtungen ermöglicht.

Es ist eine weitere Aufgabe der vorliegenden Erfindung eine Messvorrichtung zur Verfügung zu stellen, die sehr einfach in Verbindung mit elektrischen, elektronischen oder laserbasierenden Sensoren und Datenerfassungs- und Datenübertragungseinrichtungen und elektronischen Datenverarbeitungseinrichtungen versehen werden kann.

Diese Aufgabe löst eine Messvorrichtung mit den Merkmalen des Patentanspruches 1 sowie Verfahren mit den Merkmalen der Patentansprüche 9 und 10.

Weitere Ausführungsvarianten ergeben sich aus den abhängigen Ansprüchen.

In den Zeichnungen ist ein Ausführungsbeispiel des Erfindungsgegenstandes dargestellt und in der nachfolgenden Beschreibung erläutert. Die Richtungsbegriffe werden in der in der Anatomie üblichen Weise verwendet. Es zeigt:
- Figur 1: ein Messgerät in der Ansicht von schräg vorne;
- Figur 2: eine Messlatte in der Ansicht von vorne;
- Figur 3: Messlatten bei Vermessung einer Schulterpartie eines Patienten;
- Figur 4: einen schematischen Schnitt durch das Messgerät in Höhe des dritten Messelementes, wobei der Schulterbereich eines Patienten in verdrehter Stellung in durchgezogener Linie dargestellt ist; und
- Figur 5: ein Datenblatt zur Aufnahme der Fussstellung eines Probanden im frei fixierten Stand.

Die erfindungsgemässe Lösung erlaubt, dass der Patient während der Messung in einer natürlichen Haltung steht, wobei die gesamte Körperhaltung im dreidimensionalen Raum berücksichtigt wird. Die erfindungsgemässe Vorrichtung weist eine Positionierhilfe auf, welche mindestens annähernd über die gesamte Körperlänge des Patienten wirkt. Diese visuelle Positionierhilfe wird durch mindestens zwei Vertikalachsen gebildet, an die sich der Patient jedoch nicht anlehnt. Vorteilhafterweise sind an den Vertikalachsen mindestens zwei, bevorzugterweise drei, vertikal verschiebbare Messelemente angeordnet.

Die erfindungsgemässe Messvorrichtung weist eine Justiereinheit auf, mittels der die zu vermessende Person in einer optimalen Position fixiert, quasi eingefroren, werden kann, ohne dass ihre natürliche Körperhaltung beeinträchtigt wird. Die zu vermessende Person wird dabei bezüglich der Vertikalachse eingemittet. In dieser Stellung wird mittels Waagen die unterschiedliche Gewichtsbelastung im statischen Mittelpunkt und mittels der Messelemente die Fehlstellungen im Bereich der Beine, des Hüfte und des Schultergürtels gemessen. Die erfindungsgemässe Vorrichtung erlaubt es jedoch auch, wie im weiteren ausgeführt wird, einen Probanden/Probandin im freien Stand zu vermessen. Dabei wird die zu vermessende Person in einer freien, das heisst nicht eingemitteten Position fixiert.

Die Doppel-Gewichtswaagen liefern klare Messwerte, anhand deren Differenz eine vorhandene Fehlbelastung einfach und für die vermessene Person einfach verständlich visualisiert werden kann.

Mittels der erfindungsgemässen Messvorrichtung lässt sich wie bereits bei der aus der AT-U-002'239 bekannten Vorrichtung feststellen, ob der Patient seinen rechten und linken Fuss beziehungsweise die entsprechenden Seiten des Beckens unterschiedlich belastet. Der Patient ist relativ unverkrampft, da ihm der Messvorgang, das heisst das Stehen auf einer Gewichts-Waage, vertraut ist. Des weiteren erlaubt die erfindungsgemässe Vorrichtung jedoch auch noch Verdrehungen von Becken, Schultergürtel, Kopf und Rückgrat zu erfassen. Auch der Grad der Abweichung der Schultern gegenüber der horizontalen kann ermittelt werden. Es können in einer bevorzugten Ausführungsform der Erfindung auch Stellungsabweichungen der Beine (umgangssprachlich O- oder X-Beine) vermessen werden.

In einer bevorzugten Ausführungsform sind die Waagen entfernbar in der Vorrichtung Dadurch lassen sich mehrere Messschritte durchführen, um so die Körperhaltung vollständig auszumessen.

Nach einer Anlernphase kann der Patient einige der Messungen selbst, ohne fremde Hilfe durchführen. Da viele falsche Körperhaltungen ohne chirurgische Eingriffe sondern lediglich mittels spezieller Gymnastikübungen korrigiert werden können, ist eine selbständige Messung von Vorteil, da der Patient eine positive Rückmeldung erfährt, welche wiederum den Therapieerfolg unterstützt. Um jedoch einen Körper genau und vollständig zu vermessen, ist Bedienungsperson nötig. Da die Messungen nur die Grundlage für eine exakte Diagnose liefern soll, muss die Bedienungsperson zwar zum genauen und reproduzierbaren Messen angelernt werden, aber nicht über besondere medizinische Kenntnisse verfügen.

In der beiliegenden Zeichnung ist ein Ausführungsbeispiel des Erfindungsgegenstandes dargestellt, welches in der nachfolgenden Beschreibung erläutert wird.

Die hier dargestellte Ausführungsform umfasst eine Basis 10 mit Doppel-Gewichtswaagen 11, 12 und eine Justier- und Messeinheit.

Die Basis 10 des Messgerätes umfasst eine rechte und eine linke Gewichtswaage 11, 12, welche nebeneinander angeordnet sind. Die Gewichtswaagen können mechanische oder elektronische Personenwaagen bekannter Bauart oder spezielle Anfertigungen sein. Bevorzugterweise zeigt jede Waage die Gewichtsbelastung in Absolutwerten an, wobei sie gegenüber einem unabhängigen Eichwertes absolut eichbar ist. Es ist jedoch auch möglich, dass in einer gemeinsamen Anzeige nur die Differenz der Gewichtsbelastungen der einzelnen Waagen angezeigt wird. In diesem Fall genügt es, dass die Waagen relativ zueinander eichbar sind. Die Eichung kann sowohl im Herstellungswerk durchgeführt werden oder, vor allem bei Relativeichungen, vom Benützer selber vorgenommen werden. In einer bevorzugten Ausführungsform sind elektronische Waagen mit einer Schnittstelle versehen, über die sie mit einer zentralen Datenerfassungs- und Auswertungseinheit verbunden werden können. Die Erfassung, Auswertung und Visualisierung der Messdaten kann zum Beispiel mit Hilfe spezieller Software auf einem Personal Computer erfolgen. Die Datenübermittlung kann über Kabel erfolgen. Sollten diese unerwünscht oder hinderlich sein, so können auch Funk- oder Infrarotschnittstellen eingesetzt werden.

Die zwei Waagen sind vorteilhafterweise in einem gemeinsamen Gehäuse in der Basis angeordnet. In einer bevorzugten Ausführungsform sind sie einzeln oder gemeinsam entfernbar in der Vorrichtung angeordnet.

Die Basis 10 weist Justiermittel zur Positionierung je eines Fusses der zu vermessenden Person auf. In dem gezeigten Ausführungsbeispiel sind dies eine zentrale, entlang der Medianebene verlaufende, Anschlagleiste 14 und eine hintere Anschlagleiste 13, welche von den Füssen der zu vermessenden Person berührt werden sollen. Die seitliche Anschlagleiste 14 ist im Bereich zwischen den zwei Gewichtswaagen 10,11 angeordnet. Die Anschlagleiste 14 ist so bemessen, dass die Füsse möglichst nahe beieinander liegen. Ihre Breite und somit der Abstand der Füsse beträgt 3-5cm, bevorzugterweise circa 4 cm.

Die oben beschriebene Doppel-Gewichtswaage 11, 12 wird in dieser Form zur Bestimmung von Fehlbelastungen des menschlichen Körpers eingesetzt. Da jeder Fuss auf einer unabhängigen Waage steht, zeigt jede Waage die Gewichtsbelastung auf den entsprechenden Fuss und damit auf die entsprechende Seite des Beckens an. Bei einer fehlerhaften Haltung weisen die zwei Waagen unterschiedliche Gewichtsbelastungen auf. Ein Fakt, der für die vermessene Person nachvollziehbar ist. Aus der unterschiedlichen Gewichtsbelastung lässt sich mit einer einfachen Umrechnungsformel die scheinbare Beinverkürzung des Patienten bestimmen. Oft genügt jedoch schon die Feststellung, dass eine Fehlbelastung vorhanden ist. Diese Messung ist einfach durchführbar und auswertbar und kann vom Patienten auch selber durchgeführt werden.

Für genauere Messungen ist die Messvorrichtung mit weiteren Mess- und Justiereinrichtungen versehen, welche die Lage einzelner Körperpunkte oder -bereiche des Patienten fixieren und somit eine genaue und reproduzierbare Messung gewährleisten.

Die Justier- und Messeinheit besteht aus mindestens zwei parallelen Vertikalstreben 20 und 20', an welchen mehrere Mess- und Fixierungselemente 21,22,23 angebracht sind. In diesem Ausführungsbeispiel sind insgesamt drei Mess- und Fixierungselemente zur partiellen Fixierung und Vermessung von insgesamt drei Körperbereichen vorhanden. Es können jedoch auch noch weitere Bereiche vermessen und fixiert werden, wobei die Messelemente im wesentlichen stets gleich aufgebaut sind. Lediglich das Element 21 zum Vermessen und Fixieren des Schenkel- und Kniebereichs weicht vom Grundaufbau ab.

Die aufrecht stehenden Vertikalstreben 20, 20' sind auf der Basis. 10 angeordnet. Die Vertikalstreben 20, 20' definieren eine hintere Ebene, welche parallel zur Frontalebene verläuft, und sind annähernd äquidistant zur Medianebene des Gerätes. Die Vertikalstreben 20, 20' wie auch die Fixierungs- und Messelemente 21,22,23 sind bevorzugterweise aus Rohren gefertigt. Ferner ist an den Vertikalstreben 20, 20' mindestens ein Einstiegsbügel 30 angebracht, dessen hinteres horizontal verlaufendes Grundelement 31 zwei seitlich vorragende Schenkel 32, 33 tragt, welche jeweils mit Handgriffen 34, 35 versehen sein können. Der Einstiegsbügel 30 kann in Vertikalrichtung entlang der Streben 20, 20' verschoben und in verschiedenen Vertikalpositionen befestigt werden. Der Bügel 30 erleichtert der zu vermessenden Person auf das Messgerät 1 zu steigen, bietet in der anfänglichen Positionierungsphase sicheren Halt und vermeidet ein Festhalten an den Mess- und Fixierungselementen 22, 23, was ohne Einstiegsbügel 30 immer wieder vorkam.

Das erste Messelement 21 dient primär der Bein- und Knie-Vermessung und Fixierung und ist zwischen Basis 10 und zweitem Messelement 22 an den Vertikalstreben 20, 20' angeordnet. Es weist einen an den Vertikalstreben 20, 20' vertikal verschiebbar gehaltenen Grundblock 210 auf, der ein Paar hintere Querstreben 211 trägt. Die Querstreben 211 tragen über ein Winkelelement 213 ein rechtwinklig zu Ihnen angeordnetes zweites Paar seitlicher Sagittalstreben 214, welche wiederum einen über ein Winkelelement 215 rechtwinklig zu Ihnen angeordneten Frontalstab 216 tragen. Aus Stabilitätsgründen sind die Streben 211 und 214 vorteilhafterweise als Doppelrohre ausgebildet. Die Streben 211 und 214 und der Stab 216 definieren und liegen in einer Horizontal- oder Transversalebene. Die Winkelelemente 213 und 215 sind so ausgebildet, dass die Sagittalstrebe 214 transversal und der Frontalstab 216 sagittal zu den Vertikalstreben 20, 20' innerhalb dieser Horizontalebene verschoben werden können. Die Verschiebbeweglichkeit ist durch die Länge der Streben 211 und 214 begrenzt. Die Winkelelemente 213, 215 weisen Mittel zum Arretieren der Streben, respektive des Frontalstabes in einer gewünschten Position auf. Streben 211, 214 sowie Frontalstab 216 sind mit einer Skalierung versehen, die es erlaubt die Relativposition der Winkelelemente 213, 215 auf den Streben 211, 214 hinlänglich genau zu ermitteln. Die Skalierung auf dem Frontalstab dient dazu die Position von im folgenden noch genauer zu beschreibenden Messlatten 50 zu ermitteln. In einer bevorzugten Ausführungsform ist die Skalierung vom Benutzer visuell ablesbar. Sollen die Daten direkt elektronisch erfasst werden, so kann die Skalierung allerdings auch in elektromagnetisch ablesbarer Form angebracht sein. Die Winkelelemente sind dann mit Leseeinrichtungen versehen, die die Relativposition des Winkelelementes auf der Strebe 211, 214 ermitteln. Dieses Messsignal kann, wie bereits bei den Waagen 11, 12 erwähnt, via Kabel oder drahtlos an eine Datenerfassungseinheit weitergegeben und/oder auf einer im Winkelelement 213, 215 integrierten Anzeige dargestellt werden. Der Grundblock 210 ist in der gewünschten Höhe mittels bekannter Arretierungsmittel, wie Schraub- und/oder Klemmverbindungen, an jeder beliebigen Vertikalposition an den Vertikalstreben 20, 20' fixierbar.

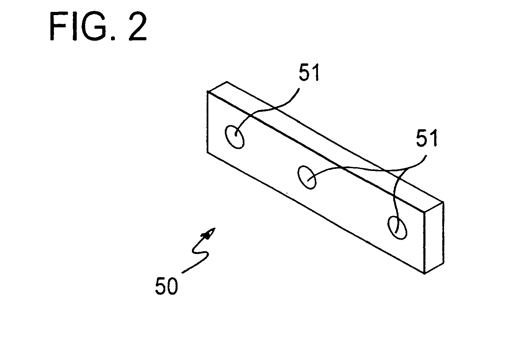

Das zweite Fixierungselement 22 ist im Gegensatz zum ersten Element 21 bilateralsymmetrisch zur Medianebene des Messgerätes 1. Analog zum Element 21 trägt auch beim Element 22 ein an den Vertikalstreben 20, 20' vertikal verschiebbar gehaltener Grundblock 220 ein Paar hintere Querstreben. An beiden Enden der Streben sind wiederum verschiebbare Winkelelemente 223 angeordnet. Die Winkelelemente tragen jeweils einen nach vorne ragenden Sagittalstab 224, 224'. Die Sagittalstäbe sind ebenfalls mit Skalierungen versehen, wie sie oben bereits beschrieben wurden und können mittels der Winkelelemente 223 mit bekannten Arretierungsmitteln in ihrer Relativlage zu den Vertikalstreben 20, 20' fixiert werden. Auf den Sagittalstäben ist jeweils mindestens eine Messplatte 50 Schwenkbeweglich und verschiebbar angebracht. Im einfachsten Fall besteht eine Messplatte 50 aus einer länglich rechteckigen Platte, wie sei in der Figur 2 dargestellt ist. In der Platte 50 sind endständig und in der Mitte je eine Bohrung 51 angebracht. Diese Bohrungen sind so bemessen, dass die Messplatte mit geringem Widerstand auf dem Sagittalstab 224 verschoben und geschwenkt werden kann und dabei genügend Klemmkraft ausübt um beim Ausbleiben von äusserer Krafteinwirkung in jeder Schwenkkposition zu verbleiben. Die Latten 50 sind vorteilhafterweise aus Kunststoff gefertigt. Die drei Bohrungen 51 erlauben es je nach Bedarf die zur Messung verfügbare Länge der Platte schnell und einfach nur durch umstecken von einem Loch 51 zum nächsten zu variieren. Es können natürlich auch Latten mit einem oder zwei Löchern Verwendung finden.

Das zweite Messelement 22 dient primär der Becken-Vermessung und Fixierung und ist im dargestellten Ausführungsbeispiel zwischen dem ersten Messelement 21 und der Einstiegshilfe 30 an den Vertikalstreben 20, 20' angeordnet. Oberhalb des zweiten Messelementes 22 kann eine bekannte Hüftwasserwaage an den vertikalen Streben 20, 20' montiert werden.

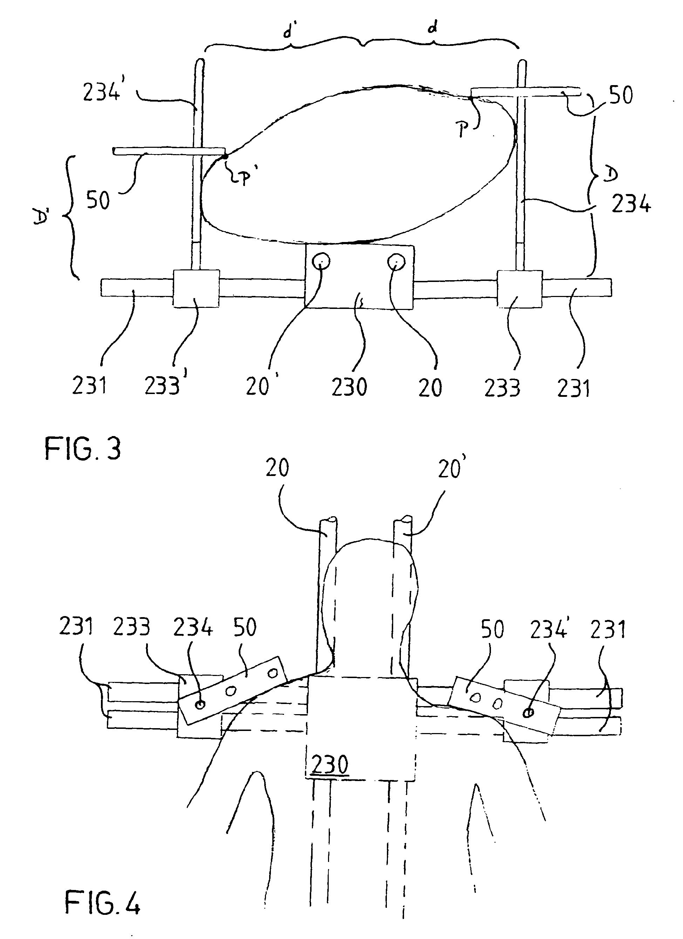

Das dritte Mess- und Fixierungselement 23 dient der Vermessung und Fixierung des Oberkörpers und ist oberhalb der Einstiegshilfe 30 an den Vertikalstreben 20, 20' angeordnet. Es weist im wesentlichen den selben Grundaufbau auf wie das zweite Messelement 22 und ist ebenfalls mit Messlatten 50 versehen. Da die Messlatten des dritten Messelementes neben der Vermessung der Distorsion des Schultergürtels auch zur Vermessung der Schulterposition dienen, sind sie mit einer Winkelmesseinrichtung versehen. Aus der Figur 4 ist ersichtlich, wie die Abweichung der Schulterposition gegenüber der Horizontalen ermittelt wird. Im einfachsten Fall kann eine Referenzlinie auf dem Sagittalstab und eine Winkelskala auf der Messplatte angebracht sein, anhand welcher der Schwenkwinkel der Messplatte bestimmt werden kann.
Zur Vermessung von Asymmetrien des Kopfes, insbesondere des Gesichts, und des Halses kann das Winkelelement 215 mit Frontalstab 216 an einer Sagittalstrebe 234 oder 234' angebracht werden.

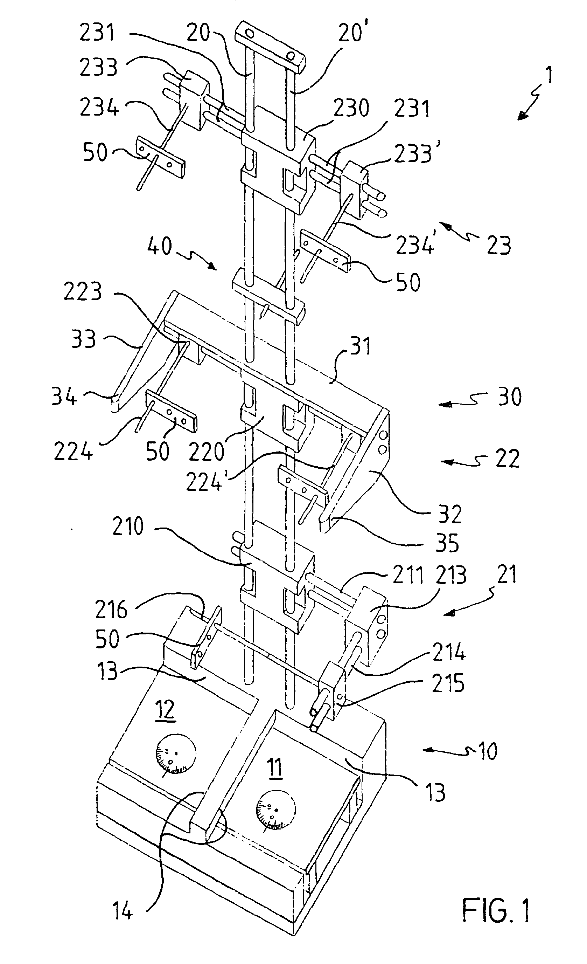

Des weiteren ist zwischen den Vertikalstreben 20, 20' ein weiteres Messelement 40 angebracht, das mindestens einen Messstift 43 zur skoliosen Ausmessung des Rückgrates trägt. Der Messstift ist transversal und sagittal verschiebbar in einem an den Streben 20, 20' in vertikaler Richtung verschiebbaren Grundblock gehalten. Der Messstift ist mit einer Skalierung versehen, so dass die Lage seiner Spitze gegenüber der von der hinteren Anschlagleiste 13 definierten hinteren Frontalebene gemessen werden kann. Die Abweichung des Rückgrates gegenüber der Medianebene kann in einer bevorzugten Ausführungsform direkt durch einen Stift ermittelt werden, der im oder am Grundblock vertikal verschiebbar gehalten ist. Diese Vertikalverschiebung wird im in der Figur 1 dargestellten Beispiel durch einen Schlitten ermöglicht, welcher den dorsoventral verschiebbaren Stift trägt. Eine Skalierung auf dem Grundblock oder Schlitten ermöglicht es die Vertikalverschiebung zu messen. Ein weiteres Messelement mit einem Stift kann oberhalb der dritten Messeinheit 23 an den Vertikalstreben 20, 20' angeordnet sein. Damit können einerseits die Halswirbelsäule und verschiedene Parameter der Kopfstellung und andererseits auch die Körpergrösse ermittelt werden.

Mindestens eine der Vertikalstreben 20, 20' ist mit einer Skala versehen deren Nullpunkt in der von den Waagenoberflächen gebildeten Standebene liegt. Die Skalierung kann vom bekannten optisch ablesbaren Typ und/oder elektromagnetisch kodiert sein. Analog zu den Winkelelementen können auch die Grundblöcke 210, 220, 230, mit digitalen Messwertanzeigen sowie mit Schnittstellen zur Datenübermittlung ausgerüstet sein. Die Vertikalposition der Messeinheiten 21, 22, 23 und 40 lässt sich jederzeit schnell und präzise ermitteln. Die Grundblöcke 210, 220, 230 und können mittels der bekannten Arretierungsmittel in einer bestimmten vertikalen Position auf den Vertikalstreben 20, 20' fixiert werden.

Im Bereich des zweiten Fixierungselementes 22 für die Hüfte ist in einer hier nicht dargestellten Ausführungsform wie bereits oben erwähnt eine Hüft-Wasserwaage lösbar angebracht. Sie besteht wie die bekannten Hüft-Wasserwaagen aus einem leicht gebogenen, stabförmigen Grundkörper und beidseitig daran angeordneten, schwenkbaren Schenkeln. Im Grundkörper ist mindestens eine Libelle zur Bestimmung der Lage der Wasserwaage gegenüber der Horizontalen enthalten. Die Schenkel sind bevorzugterweise länger als diejenigen der bekannten Hüft-Wasserwaagen, damit sie auch bei korpulenten Personen bis in den vorderen Bereich der Hüften ragen können, obwohl die Hüftwasserwaage nicht an den Körper des Patienten gepresst wird. Die Hüft-Wasserwaage ist zum Beispiel oberhalb des zweiten Messelementes 22 an den Vertikalstreben 20, 20' verschiebbar und arretierbar gehalten. Sie kann jedoch auch am zweiten Grundblock 220 selber angeordnet und mit diesem gemeinsam verschiebbar sein.

Ferner kann im obersten Bereich der Vertikalstreben 20, 20' eine verschiebbare Messlatte zur Bestimmung der Körpergrösse angeordnet sein, wobei der an den Vertikalstreben 20, 20' angebrachte Massstab genutzt wird. Die Messung der Grösse dient unter anderem dem Nachweis, dass der Patient nach Korrektur der Fehlhaltung aufrechter steht und somit grösser erscheint.

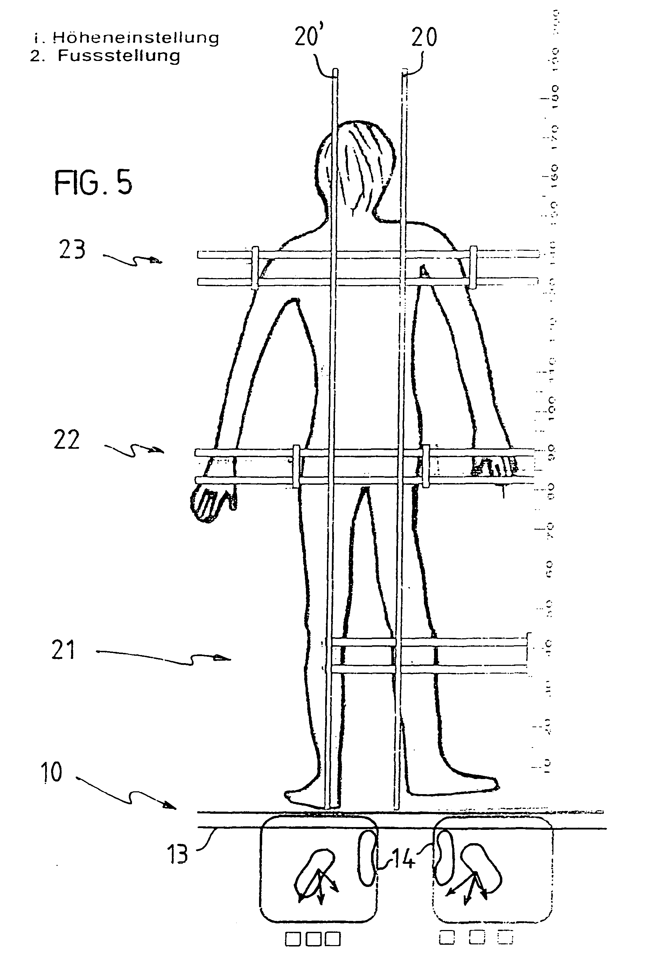

Im folgenden soll das erfindungsgemässe Meßverfahren näher beschrieben werden. Eine schnelle Aufnahme, die von einer geübten Person im ca. 5 Minuten durchgeführt werden kann umfaßt beispielsweise folgende Schritte:
1. Zur Durchführung der Messung steht die zu vermessende Person mit dem Rücken zu den Vertikalstreben 20, 20' frei auf der Basis 10, respektive der Doppel-Gewichtswaage 11, 12. Der rechte Fuss wird auf die rechte Gewichtswaage 12 und der linke auf die linke Gewichtswaage 11 gestellt. Die Meß- und Fixierungselemente 21, 22, 23 werden, angepasst auf die Grösse des Probanden, auf ungefähre Knie-, Hüft- und Schulterhöhe eingestellt.
2. Die Fußstellung im freien Stand wird wie in Figur 5 dargestellt aufgezeichnet und die Rotation der Sohle wird gemessen. Die Figur 5 ist einem ersten Datenblatt aus einer Serie von Datenblättern nachempfunden, das bei einer tatsächlichen Messung zum schriftlichen erfassen der Messergebnisse verwendet werden kann. Die Datenblätter dienen gleichzeitig als Wegleitung durch eine Messung, da sie sicherstellen, dass keine Messschritte vergessen werden.
   Aus dem freien Stand heraus werden wie ebenfalls in der Figur 5 angedeutet die Füsse an den Anschlägen 13, 14 positioniert. Nun wird der Proband in dieser freien Stellung oder Haltung mittels der Sagittalstreben 224, 224', 234, 234' fixiert. Der Proband berührt dabei mit dem rückwärtigen Gesäss- und Schulterbereich die Grundplatten 220 und 230 oder Teile der Horizontalstreben 231, ohne sich an einer Vertikalstrebe einmitten zu können.
3. die laterale Stellung der Hüfte wird aufgezeichnet
4. die laterale Stellung der Schultern wird aufgezeichnet
5. der Beindruck wird von beiden Waagen abgelesen
6. die laterale Abweichung der Kopfstellung wird gemessen
7. die laterale Abweichung der Halsstellung wird gemessen
8. die Schulterstellung wird gemäss Figur 4 ermittelt.
   Nun werden Schultern und Hüfte zentriert und die Knie werden mit dem Frontalstab des ersten Fixierungselementes in einer gestreckten Stellung fixiert. In dieser zentrierten Stellung kann der Beckenschiefstand gemessen werden. Es wird dazu oberhalb des zweiten Messeelementes 22 eine Hüftwasserwaage an den Vertikalstreben 20, 20' befestigt und die scheinbare Beinverkürzung wird in bekannter Weise ermittelt.
   Im Gegensatz zu der oben beschriebenen schnellen oder Kurz-Aufnahme werden bei einer ausführlichen oder "großen" Aufnahme, die etwa 15 Minuten dauert, wesentlich mehr Messungen durchgeführt und mehr Messpunkte und Masse aufgenommen. Anfänglich steht der Proband wiederum frei im Meßgerät und es werden die Messungen gemäß der obigen Punkte 1 bis 7 durchgeführt. Zusätzlich werden daran anschliessend vorteilhafterweise noch folgende Messungen durchgeführt:
9. Handstellung und
10. Fußlängen-Differenz werden ermittelt. Für die Ermittlung von etwaigen Verkrümmungen der Beine, zum Beispiel:
11. O Beinen
12. X-Beine und der
13. Schenkelabweichung
14. Knieabweichung
15. Wadenabweichung
   wird das Messelement 21 jeweils in den gewünschten Knie-, Ober- oder Unterschenkelbereich verschoben. Mittels der einen oder mehrerer Messlatten 50, welche auf dem Frontalstab 216 verschoben und nach hinten geschwenkt werden können, werden dazu die Masse der Abweichung von der Medianebene innen und aussen an den Schenkel genommen.
   In weiter Messungen werden ermittelt:
16. Kniescheibenhöhe
17. Größe
18. Kopfhaltung
19. Hals
20. Schulterblatt unten oder Brusthöhe
21. Lenden
22. Gesäss
23. Kniegelenk
24. Kniegelenkdefizit
25. Lot Ohr-Hüfte-Fußknöchel
26. Schulterrotation, wie in der Figur 3 dargestellt.
   Bei dieser Messung kommen nun die Messlatten 50 auf den Saggitalstäben 234, 234' zum Einsatz. Die Entfernungen D, D' der Messpunkte von der hinteren Frontalebene, welche durch Anschlag 13 definiert ist, wird ermittelt. Diese Messung, respektive diese Messwerte schaffen in Kombination mit den Werten aus den Lateralpositionen der Saggitalstäbe 234, 234' eine korrekte Grundlage um die Rotation der Schultern zu ermitteln. Im dargestellten Beispiel sind die herkömmlich ermittelten lateralen Abstände der Schultern D, D' von der Medianebene annähernd identisch. Im herkömmlichen Messverfahren mit herkömmlichen Geräten würde werde eine Asymmetrie noch eine Rotation erkannt. Erst der Einsatz der Messlatten 50 ermöglicht es, die Rotation zu erkennen und genau zu vermessen.
   Zum ermitteln der
27. Beckenrotation werden die Messlatten 50 auf den Sagittalstäben 224, 224' in analoger Weise verwendet.
28. Beckenstellung
29. Handstellung
   Phaenologisch werden im weiteren die Fuße auf folgende Abnormitäten beurteilt:
30. Hammerzehen
31. Halux
32. Spreizfuß
33. Plattfuß
34. Knickfuß
35. Senkfuß
   Nun werden wiederum Schulter und Hüfte zentriert und der Kniebalken 216 angesetzt, um den Beckenschiefstand mittels einer Hüft-Wasserwaage messen zu können.

Bei einem etwaigen Beckenschiefstand wird in bekannter Art und Weise die Fußsohle des kürzeren Beines mit Plättchen unterlegt. Vorteilhafterweise sind für jeweils eine oder mehrere Meßfolgen Datenaufnahme-Blätter vorbereitet in die die jeweiligen Meßergebnisse direkt eingetragen werden können. Wie bereits erwähnt erleichtern es diese Datenblätter einem ungeübten Benutzer die Messungen in der richtigen Reihenfolge durchzuführen und verhindern, dass einzelne Messungen vergessen werden. Die Messwerte können anhand der Blätter ausgewertet und für die Diagnose verwendet werden, oder sie können auch in einem Computer eingegeben und dort weiterverarbeitet werden. Natürlich ist es auch möglich die Daten direkt in einem Computer aufzunehmen.

Die erfindungsgemässe Vorrichtung ermöglichte es Patienten verschiedenste Fehlstellung selbst zu ermitteln. Die Vorrichtung erlaubt es also dem Patienten Therapiefortschritte selbst oder mit Hilfe einer weiteren Person zu ermitteln und zu kontrollieren. Wird zum Beispiel in einer Langzeittherapie versucht Muskelverspannungen, weiche zu Fehlstellung führen, zu lösen, so kann der Patient den Therapieerfolg selbst überprüfen und wird bei erfolgreicher Therapie durch die positive Rückmeldung nicht nur informiert, sondern auch für die weitere Therapie motiviert.

Die Rotation des Beines, des Beckens, die Hürtverschiebung, und der Beckenhochstand verursachen verschiedene Ausweich- und Kompensationshaltungen des Ober- zum Unterkörpers. In den bisher bekannten zweidimensionalen Meßverfahren werden solche Rotationen als Beinverkürzungen gemessen, obwohl die Extremitäten, respektive die Beinknochen in keiner Weise verkürzt sind. Erst das genaue Erfassen der Abweichung entlang aller drei Raumachsen und in Beziehung zu einem statischen Mittelpunkt ermöglicht eine exakte Diagnose und darauf basierend eine korrekte Therapie.

## Patentansprüche

1. Messvorrichtung (1) zur Bestimmung einer Fehlhaltung und/oder Fehlbelastung des menschlichen Körpers in aufrechter Haltung, mit einem zwei Waagen (12, 11) aufweisenden Basiselement (10) und mit einer Justiereinheit zur Positionierung einzelner Körperpunkte oder - bereiche der zu vermessenden Person, wobei die Justiereinheit (2) zweite und dritte vertikal verschiebbare Fixierungselemente (22,23) zur Fixierung von Hüft- und Schulterbereich aufweist, wobei jedes Fixierungselement (22,23) verschiebbare Querstreben (231) und daran verschiebbar befestigbare Sagittalstäbe (224, 234) aufweist, wobei am Basiselement Anschläge (13, 14) zur Positionierung der Fersen der zu vermessenden Person vorhanden sind, die eine hintere Frontalebene definieren,
**dadurch gekennzeichnet, dass** mindestens zwei Vertikalstreben (20, 20') eine Positionierachse zur Definition einer mittleren Körperlinie der zu vermessenden Person definieren und die Fixierungselemente tragen, die Mess- und Fixierungselemente (22, 23) sind, die es erlauben die Lage von Körperpunkten in Frontal-, Transversal- und Sagittalebenen zu ermitteln, wobei auf den Sagittalstäben (224, 224' 234, 234') entlang der Stabachsen verschiebbare Messlatten (50) schwenkbeweglich angebracht sind, mittels derer im Zusammenwirken mit den Querstreben (231) und den Sagittalstäben (224, 224', 234, 234') die Position eines zu vermessenden Körperpunktes in einer von Querstreben (231) und Sagittalstäben (224, 224', 234, 234') definierten Transversalebene bestimmbar ist.

2. Messvorrichtung (1) nach Anspruch 1, **dadurch gekennzeichnet, dass** ein erstes Fixier- und Messelement (21) zum Fixieren und Vermessen der Knieregion Querstreben (211) und daran verschiebbar befestigbare Sagittalstreben (214) aufweist, die einen Frontalstab (216) tragen und entlang der Stabachse mindestens eine verschiebbare Messlatte (50) schwenkbeweglich angebracht ist.

3. Messvorrichtung nach Anspruch 2, **dadurch gekennzeichnet, dass** die Querstreben (211, 231) von je einem Grundblock (210, 220, 230) verschiebbar befestigbar an den Vertikalstreben (20, 20') gehalten sind und die Sagittalstreben (214) und Sagittalstäbe (224, 224', 234, 234') von je einem Winkelelement (213, 223, 223', 233, 233') verschiebbar befestigbar an den Querstreben (211, 231) gehalten sind.

4. Messvorrichtung nach einem der Ansprüche 2 bis 3, **dadurch gekennzeichnet, dass** die Messlatten (50) mit mindestens einer endständigen Bohrung (51) zur Aufnahme des tragenden Frontal- (216) oder Sagittalstabes (224, 224', 234, 234') versehen ist, wobei jeweils der Stab (216, 224, 224', 234, 234') mit einer Referenzlinie und die Messplatte (50) mit einer die Bohrung (51) umgebenden Winkelskala versehen ist.

5. Messvorrichtung nach einem der Ansprüche 2 bis 4, **dadurch gekennzeichnet, dass** mindestens je eine der Vertikal- (20, 20') und Sagittalstreben (214) und die Sagittal- (224, 224', 234, 234') und Frontalstäbe (216) mit einer Längenskala versehen sind.

6. Messvorrichtung nach Anspruch 5, **dadurch gekennzeichnet, dass** die Längenskalen visuell und/oder elektromagnetisch ablesbar sind.

7. Messvorrichtung nach Anspruch 6, **dadurch gekennzeichnet, dass** Grundblöcke (210, 220, 230) und/oder Winkelemente (213, 215, 223, 223', 233, 233') mit optischen und/oder elektronischen Leseeinrichtungen zum Ablesen der Längenskalen versehen sind.

8. Messvorrichtung nach Anspruch 1, **dadurch gekennzeichnet, dass** an den Vertikalstreben (20, 20') im Bereich oberhalb des zweiten Mess- und Fixierelementes (22) eine Einstiegshilfe (30) befestigbar ist.

9. Verfahren zur Bestimmung einer Fehlbelastung des menschlichen Körpers mittels der Messvorrichtung gemäss einem der Patentansprüche 1 bis 8, **dadurch gekennzeichnet, dass**
a) in einem ersten Schritt die Füsse eines frei stehenden Probanden an den Anschlägen (13, 14) positioniert werden und
b) in einem weiteren Schritt der Proband in dieser freien Stellung oder Haltung mittels der Sagittalstäbe (224, 224', 234, 234') im Hüft- und Schulterbereich fixiert wird, und
c) in einem nachfolgenden Schritt die Rotation im Hüft- und Schulterbereich anhand von Messpunkten (P, P'), die mittels der Messlatten (50) ermittelt werden, vermessen wird.

10. Verfahren zur Bestimmung einer Fehlbelastung des menschlichen Körpers mittels der Messvorrichtung gemäss einem der Patentansprüche 1 bis 8,
**dadurch gekennzeichnet, dass**
a) in einem ersten Schritt die Füsse eines frei stehenden Probanden an den Anschlägen (13, 14) positioniert werden und
b) in einem weiteren Schritt der Proband in dieser freien Stellung oder Haltung mittels der Sagittalstäbe (224, 224', 234, 234') im Hüft- und Schulterbereich fixiert wird, und
c) in einem nachfolgenden Schritt die Abweichung des Rückgrates gegenüber der Medianebene mittels einem Stift ermittelt wird.

## Claims

1. A measurement device (1) for determining an incorrect posture and/or incorrect loading of the human body in the upright posture, with a base element (10) comprising two scales (12, 11) and wth an adjustment unit for positioning individual body points or body regions of the person to be measured, wherein the adjustment unit (2) comprises second and third vertically displaceable fixation elements (22, 23) for fixing the hip- and shoulder region, wherein each fixation element (22, 23) comprises displaceable cross-members (231) and sagittal rods (224, 234) displaceably fastenable thereon, wherein abutments (13, 14) for positioning the heels of the persor to be measured and which define a rear frontal plane are present on the base element,
**characterised in that** at least two vertical members (20, 20') define a positioning axis for the definition of a middle body line of the person to be measured and carry the fixation elements which are measurement- and fixation elements (22, 23) which permit the position of body points in the frontal-, transversal- and sagittal planes to be determined, wherein measurement bars (50) displaceable along the rod axes are attached on the sagittal rods (224, 224', 234, 234') in a pivotally movable manner, by way of which, in cooperation with the cross-members (231) and the sagittal rods (224, 224', 234, 234'), the position of a body point to be measured in a transversal plane defined by cross-members (231) and sagittal rods (224, 224', 234, 234') may be determined.

2. A measurement device (1) according to claim 1, **characterised in that** a first fixation- and measurement element (21) for fixing and measurement of the knee region comprises cross-members (211) and sagittal members (214) which are displaceably fastenable thereon and which carry a frontal rod (216), and at least one displaceable measurement bar (50) is attached in a pivotally movable manner along the rod axis.

3. A measurement device according to claim 2, **characterised in that** the cross-members (211, 231) are held on the vertical members (20, 20') in a displaceably fastenable manner by in each case a base block (210, 220, 230), and the sagittal members (214) and the sagittal rods (224, 224', 234, 234') are held on the cross-members (211, 231) is a displaceably fastenable manner by in each case one angle element (213, 223, 223', 233, 233').

4. A measurement device according to one of the claims 2 to 3, **characterised in that** the measurement bars (50) are provided with at least one bore (51) at the end, for receiving the carrying frontal rad (216) or sagittal rod (224, 224', 234, 234'), wherein in each case the rod (216, 224, 224', 234, 234') is provided with a reference line, and the measurement bar (50) is provided with an angular scale surrounding the bore (51).

5. A measurement device according to one of the claims 2 to 4, **characterised in that** at least in each case one of the vertical members (20, 20') and sagittal members (214) and the sagittal rods (224, 224', 234, 234') and frontal rods (216) are provided with a linear scale.

6. A measurement device according to claim 5, **characterised in that** the linear scales may be read off visually and/or electromagnetically.

7. A measurement device according to claim 6, **characterised in that** base blocks (210, 220, 230) and/or angle elements (213, 215, 223, 223', 233, 233') are provided with optical and/or electronic read devices for reading off the linear scales.

8. A measurement device according to claim 1, **characterised in that** an accessing aid (30) is fastenable on the vertical members (20, 20') in the region above the second measurement-and fixation element (22).

9. A method for determining an incorrect loading of the human body by way of the measurement device according to one of the patent claims 1 to 8, **characterised in that**
a) in a first step, the feet of a freely standing test person are positioned on the abutments (13, 14) and
b) in a further step, the test person is fixed In this free position or posture in the hip- and shoulder region by way of sagittal rods (224, 224', 234, 234') and
c) in a subsequent step, the rotation in the hip- and shoulder region is measured by way of measurement points (P, P') which are determined by way of the measurement bars (50).

10. A method for determining an incorrect loading of the human body by way of the measurement device according to one of the patent claims 1 to 8, **characterised in that**
a) in a first step, the feet of a freely standing test person are positioned on the abutments (13, 14) and
b) in a further step, the test person is fixed in this free position or posture is fixed in the hip and shoulder region by way of the sagittal rods (224, 224', 234, 234') and
c) in a subsequent step, the deviation of the backbone with respect to the median plane is determined by way of a pin.

## Revendications

1. Dispositif de mesure (1) pour la détermination d'une posture incorrecte et/ou d'une charge incorrecte du corps humain en une posture droite, ayant un élément de base (10) comprenant deux balances (12, 11) et une unité d'ajustage pour le positionnement de points isolés du corps ou de zones isolées du corps de la personne à mesurer, l'unité d'ajustage (2) comprenant des deuxième et troisième éléments de fixation (22, 23) déplaçables verticalement pour la fixation de la zone des hanches et des épaules, chaque élément de fixation (22, 23) comprenant des entretoises transversales déplaçables (231) et, déplaçables par rapport à ces dernières, des barres sagittales fixables (224, 234), des butées (13, 14) étant disponibles sur l'élément de base pour le positionnement des talons de la personne à mesurer, qui définissent un plan frontal arrière,
**caractérisée en ce qu'**au moins deux entretoises verticales (20, 20') définissent un axe de positionnement pour la définition d'une ligne du corps moyenne de la personne à mesurer et portent les éléments de fixation, qui sont des éléments de mesure et de fixation (22, 23), qui permettent d'évaluer la situation des points du corps dans les plans frontal, transversal et sagittale, des lattes de mesures (50) déplaçables étant montées mobiles en rotation sur les barres sagittales (224, 224', 234, 234') le long des axes des barres, lattes de mesures au moyen desquelles, avec la coopération des entretoises transversales (231) et des barres sagittales (224, 224', 234, 234'), la position d'un point du corps à mesurer est déterminable dans un plan transversal défini par les entretoises transversales (231) et les barres sagittales (224, 224', 234, 234').

2. Dispositif de mesure (1) selon la revendication 1, **caractérisée en ce qu'**un premier élément de mesure et de fixation (21) pour la fixation et la mesure de la région du genou comprend des entretoises (211) et, déplaçables par rapport à ces dernières, des entretoises sagittales fixables (214), qui portent une barre frontale (216) et le long de l'axe de la barre est montée mobile en rotation au moins une latte de mesure déplaçable (50).

3. Dispositif de mesure (1) selon la revendication 2, **caractérisée en ce que** les entretoises transversales (211, 231) sont maintenues chacune par un bloc de base déplaçable et fixable aux entretoises verticales (20, 20') et les entretoises sagittales (214) et les barres sagittales (224, 224', 234, 234') sont maintenues chacune par un élément d'angle (213, 223, 223', 233, 233') déplaçable et fixable aux entretoises transversales (211, 231).

4. Dispositif de mesure (1) selon l'une des revendications 2 à 3, **caractérisée en ce que** les lattes de mesure (50) sont munies d'au moins un perçage d'extrémité (51) pour l'admission de la barre sagittale (224,224',234,234') ou frontale (216) porteuse, chaque barre (216, 224,224',234,234') étant munie d'une ligne de référence et la plaque de mesure (50) étant munie d'une graduation d'angle entourant le perçage (51).

5. Dispositif de mesure (1) selon l'une des revendications 2 à 4, **caractérisée en ce qu'**au moins une des entretoises verticales (20, 20') et une des entretoises sagittales (214) et les barres sagittales (224, 224', 234, 234') et frontales (216) sont munies d'une graduation de longueur.

6. Dispositif de mesure (1) selon la revendication 5, **caractérisée en ce que** les graduations de longueurs sont lisibles visuellement et/ou électro-magnétiquement.

7. Dispositif de mesure (1) selon la revendication 6, **caractérisée en ce que** les blocs de base (210, 220, 230) et/ou les éléments d'angles (213, 215, 223, 223', 233, 233') sont munis d'équipements de lecture optique et/ou électronique pour la lecture des graduations de longueurs.

8. Dispositif de mesure (1) selon la revendication 1, **caractérisée en ce que** une aide d'accès est fixable aux entretoises verticales (20, 20') dans la zone au-dessus du deuxième élément de mesure et de fixation (22).

9. Procédé pour la détermination d'une charge incorrecte du corps humain au moyen du dispositif de mesure selon l'une des revendications 1 à 8, **caractérisé en ce que**
a) dans une première étape, les pieds d'un participant se tenant librement debout sont positionnés contre les butées (13, 14) et
b) dans une étape ultérieure, le participant est fixé dans cette position ou posture libre au moyen des barres sagittales (224, 224', 234, 234') dans la zone des hanches et des épaules, et
c) dans une étape suivante, la rotation dans la zone des hanches et des épaules est mesurée à l'aide de points de mesure (P, P'), qui sont évalués au moyen des lattes de mesures (50).

10. Procédé pour la détermination d'une charge incorrecte du corps humain au moyen du dispositif de mesure selon l'une des revendications 1 à 8, **caractérisé en ce que**
a) dans une première étape, les pieds d'un participant se tenant librement debout sont positionnés contre les butées (13, 14) et
b) dans une étape ultérieure, le participant est fixé dans cette position ou posture libre au moyen des barres sagittales (224, 224', 234, 234') dans la zone des hanches et des épaules, et
c) dans une étape suivante, la déviation de la colonne vertébrale par rapport au plan médian est évalué au moyen d'un crayon.
